# EUROPEAN PATENT APPLICATION

(11) **EP 0 865 801 A2**
(43) Date of publication of application: **23.09.1998**
(21) Application number: 98104330.0
(22) Date of filing: 11.03.1998
(51) Int. Cl.: A61N 1/14, A61N 1/16

(54) **Watch-straps, belts, arm-bands or the like and/or animal collars with means for facilitating the flow of electromagnetic or electrostatic charges**

(30) Priority: 21.03.1997 IT VR970013 U
(71) Applicant: Cecchini S.r.l., 37015 Domegliara (VR) (IT)
(72) Inventor: Cecchini, Giovanni, 37015 Domegliara (Domegliara) (IT)
(74) Representative: Savi, Alberto

(57) **Abstract**

This is a proposal for using copper as a copper insert to be put inside a strap, especially a watch-strap, or more generally inside a belt to be worn for instance in connection with a particular article of clothing, or inside an animal collar, with all the consequent beneficial effects.

In general, the peculiarity of the present invention consists in introducing at least a copper insert (4) between an outer covering (2) and an inner lining (3) of the above indicated strap.

The described assembly involves that prominent parts (6) of the copper insert (4) touch the skin of a person using such a watch-strap or the like. In this way, the skin of the user is connected with the whole copper insert itself, there being beneficial reactions owing to a favourable exchange of electromagnetic or electrostatic flows.

## Description

The present invention relates to watch-straps, arm-bands, belts or the like and/or animal collars the peculiarity of which is the insertion of means for facilitating the flow of electromagnetic or electrostatic charges.

As it is known, there is a great variety of solutions in the field of watch-straps or belts in general as accessories for clothing. These solutions are based on the visual appearance to be achieved and on the practicalness as regards the application.

Belts and watch-straps consist in general of parts to be reciprocally buckled. These parts are formed by an outer covering in leather or the like and an inner lining, the former and the latter being sewn together. One of the two ends of such parts is provided with a buckle while the other opposite end is provided with suitable holes for permitting the buckle to be hooked.

It is also known that benefits are obtained by using some metals, in particular copper metals, as an element to be put in touch with the skin, such an element being formed and shaped for instance like an arm-band, a collar or the like.

As a matter of fact, it was found that a direct touch of a copper element with the skin of a person generates beneficial reactions which are caused mainly by a favourable exchange of electrostatic or electromagnetic flows which free the person from many of the harmful tensions of electric nature which surround the person in an imperceptible way and are often, in time, one of the causes of indispositions or even diseases to prejudice of the body.

The aim of the present invention is a proposal for using copper as a copper insert to be put inside a strap, in particular a watch-strap, or more generally inside a belt to be worn for instance in connection with some articles of clothing, with all the consequent beneficial effects.

Furthermore, the said copper insert may be used in connection with animal collars, or other elements of various kinds, for instance cases for musical instruments, wind instruments or not, briefcases, magnetic band card holders, computer diskette holders, bands, compact discs, or the like.

An immediate advantage deriving from the application of the copper insert in question consists in the fact that the use of such copper inserts inside the above-mentioned accessories avoids both a visual unaesthetic appearance of the metal and a rapid oxidation or deterioration of the metal itself, there being also all the advantages deriving from the contact of copper with the body of the person.

All the above mentioned aims and advantages are reached by the present invention through watch-straps, belts, arm-bands or the like and animal collars having means for facilitating the flow of electromagnetic or electrostatic charges, characterized by the fact of being provided with means that are preferably made in copper and/or alloy copper and consist of a lamellar body and a prominent part; the said lamellar body is positioned and fixed between the outer covering and the inner lining of a watch-strap or the like, the prominent part being projecting from the said inner lining of the strap so as to permit a contact with the skin of the user preferably.

Further features and details of the present invention will better appear from the following description of the preferred embodiment which is represented as an example not restricting the invention in the accompanying drawing wherein:
- Figure 1 shows a schematic side view of a watch-strap from which it appears the components thereof before the assembly;
- Figure 2 shows a schematic side view of the same after the assembly of the components;
- Figure 3 shows a schematic view of a watch-strap as actually used when slipped on the pulse.

With reference to the accompanying drawings, number 1 refers in general to a strap or belt, preferably a watch-strap, or an arm-band, an animal collar and the like, according to the present invention.

The watch-strap consists in general of an outer covering 2 to be coupled with an inner lining 3, for instance by sewing.

The inner lining 3 touches the skin.

The peculiarity of the present invention consists in putting at least a copper insert 4 between the outer covering 2 and the inner lining 3 of the watch-strap.

More precisely, the copper insert 4 in question is formed by an oblong, laminar copper element 5 and a prominent copper part 6 which is connected with the laminar element 5. The prominent copper part 6 may be, for instance, round or may have another suitable shape.

The laminar element is made with such dimensions so as to be laterally and longitudinally included in the dimensiones of the watch-strap. Accordingly, the laminar element is entirely comprehended in the watch-strap.

The inner lining 3 of the watch-strap is provided with an opening the shape and dimensions of which correspond to the ones of the prominent part of the copper insert 6.

The assembly of the above-described watch-strap involves, therefore, that the copper insert 4 be positioned between the outer covering 2 and the inner lining 3 in such a way that the prominent part 6 comes out from the opening of the lining. The outer part is sewn on the inner part so that both parts are mutually fixed.

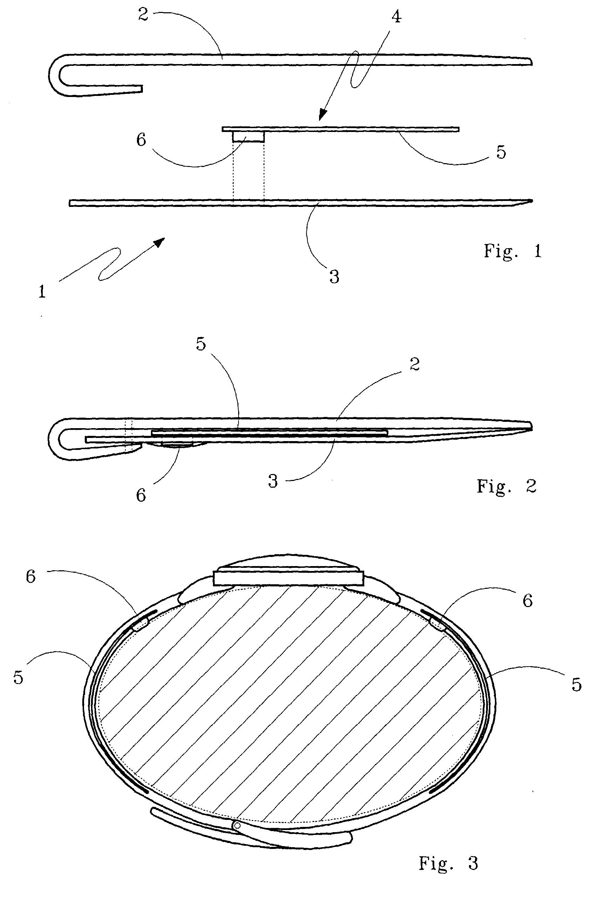

As it appears from Figure 3, the described assembly involves that the prominent parts 6 of the copper insert touch the skin of the person using such a strap. Accordingly, the skin is in contact with the whole copper insert and the person enjoys all the benefits deriving from this contact.

As an advantage, the copper insert 4 may be blocked in the strap by means of one or more coats of glue in order to prevent the copper insert 4 from any possible change of position.

Another advantage is the possibility of using this type of insert also for other objects such as belts, arm-bands, or other similar accessories, the same results as described above being obtained.

As previously mentioned, there is the possibility of using the copper insert in question on an animal collar or other articles of various kind such as cases for musical instruments, wind instruments and not, document cases, magnetic band card cases, computer floppy disc cases, bands, compact discs, or the like.

The straps or belts in question, the arm-bands or the animal collars, provided with means for facilitating the flow of electromagnetic or electrostatic charges, have been described and represented according to the preferred solution.

However, other variants may be provided. These variants are technically equivalent to the described parts and components and therefore, they are to be considered included in the scope of protection of the present invention.

## Claims

1. Watch-straps, belts or the like, arm-bands and/or animal collars, provided with means for facilitating a flow of electromagnetic or electrostatic charges, characterized by the fact of comprising means (4) that are preferably made in copper and/or alloy copper, such means being formed by a laminar body (5) and a prominent part (6); the said laminar part (5) being positioned and fixed between an outer covering (2) and an inner lining (3) of a strap or an arm-band or an animal collar or the like; the said prominent part (6) coming out from the inner lining (3) of the strap in order to permit the contact with the skin of the user.

2. Watch-straps or belts as claimed in claim 1, characterized by the fact that the said copper insert (4) may be blocked in position by means of one or more coats of glue in order to prevent the coppert insert from any possible change of position.

3. Watch-straps or belts as claimed in the preceding claims, characterized by the fact that the said copper insert (4) may be used for an animal collar or other articles of various kind, represented for instance by cases such as cases for for musical instruments, wind instruments and not, document cases, magnetic band card cases, computer floppy disc cases, bands, compact discs, or the like.
